# EUROPEAN PATENT SPECIFICATION

(11) **EP 2 427 105 B1**
(45) Date of publication and mention of the grant of the patent: **26.06.2019**
(21) Application number: 10717393.2
(22) Date of filing: 03.05.2010
(51) Int. Cl.: A61B 5/04, A61B 5/0464, G06K 9/00, G06F 19/00

(54) **ADJUDICATION OF ARRHYTHMIA EPISODE DATA SYSTEMS AND METHODS**
SYSTEME UND VERFAHREN ZUR ZUWEISUNG VON ARRHYTHMIE-EPISODENDATEN
SYSTÈMES ET PROCÉDÉS D'EXAMEN DE DONNÉES D'ÉPISODE D'ARYTHMIE

(30) Priority: 04.05.2009 US 175232 P; 30.04.2010 US 771103
(43) Date of publication of application: 14.03.2012
(73) Proprietor: Cardiac Pacemakers, Inc., St. Paul, Minnesota 55112 (US)
(72) Inventor: DONG, Yanting, Shoreview Minnesota 55126 (US); LI, Dan, Shoreview, Minnesota 55126 (US); PERSCHBACHER, David, L., Coon Rapida, Minnesota 55448 (US); ZHANG, Shijie, Cleveland, Ohio 44106 (US); MAHAJAN, Deepa, Circle Pines, Minnesota 55014 (US); PATANGAY, Abhilash, Inver Grove Heights, Minnesota 55076 (US); SHARMA, Arjun, St. Paul, Minnesota 55102 (US)
(74) Representative: Pfenning, Meinig & Partner mbB
(86) International application number: PCT/US2010/033331
(87) International publication number: WO 2010/129447

(56) References cited:
- WO-A1-2006/039693
- US-A1- 2003 204 215
- US-A1- 2004 111 119
- US-A1- 2007 112 276
- US-A1- 2007 219 456
- US-A1- 2008 281 367
- US-B1- 6 301 503
- ARIAS M A ET AL: "A single implantable cardioverter-defibrillator shock unmasking an electrical storm of 389 ventricular tachycardia episodes triggering device therapies" AMERICAN JOURNAL OF EMERGENCY MEDICINE, CENTRUM PHILADELPHIA, PA, US LNKD- DOI:10.1016/J.AJEM.2008.03.006, vol. 26, no. 9, 1 November 2008 (2008-11-01), pages 1066.E1-1066.E3, XP025583182 ISSN: 0735-6757 [retrieved on 2008-10-27]

## Description

### TECHNICAL FIELD

This disclosure relates generally to medical systems and, more particularly, to medical systems that can be used to communicate with implanted medical devices, amongst other things.

US 2007/112276 A1 discusses methods, systems, and computer program products differentiating between arrhythmic events having different origins. An implantable medical device detects the arrhythmic events. At least one method involves receiving electrograms representing arrhythmic events and sorting the arrhythmic events into groups based on similarities between the electrograms and/or differences between the electrograms. The method also involves identifying an exemplary arrhythmic event from each group to represent the group from which the exemplary arrhythmic event is identified and distinguishing each exemplary arrhythmic event via an interface display.

US 2003/0204215 A1 discusses a method and apparatus for automatically identifying various types of cardiac and non-cardiac oversensing. EGM data, including time intervals between sensed and paced events and signal morphologies, are analyzed for patterns indicative of various types of oversensing, including oversensing of far-field R-waves, R-waves, T-waves, or noise associated with electromagnetic interference, non-cardiac myopotentials, a lead fracture, or a poor lead connection. Identification of oversensing and its suspected cause are reported so that corrective action may be taken.

US 2004/0111119 A1 discusses systems and methods for improving the specificity of discriminating between a monomorphic tachyarrhythmia and a polymorphic tachyarrhythmia that examine frequency content and baseline information of the EGM as discriminatory signatures. Particular algorithms are employed to determine frequency content of the QRS complexes and are titled the Slope Distribution Metric (SDM) algorithm and the Slope Distribution Composite (SDC) algorithm.

WO 2006/039693 A1 discusses cardiac monitoring and/or stimulation methods and systems that provide monitoring, diagnosis, and defibrillation and/or pacing therapies. A signal processor receives a plurality of composite signals associated with a plurality of sources, performs a source separation, and produces one or more cardiac signal vectors associated with all or a portion of one or more cardiac activation sequences based on the source separation. A method of signal separation involves detecting a change in a characteristic of the cardiac signal vector relative to a baseline. One or more vectors and/or activation sequences may be selected, and information associated with the vectors and/or activation sequences may be stored and tracked.

US 6,301,503 B1 discusses a medical device system and method of plotting symbols representing complexes of selected arrhythmic events on an interactive display screen, for organizing, displaying and interacting with a patient's recorded arrhythmia episodes. Stored arrhythmic episode are selected from a plurality of arrhythmic episodes, where the stored arrhythmic episode having a plurality of complexes. A similarity value and a dissimilarity value is calculated for each complex of the plurality of complexes of the selected arrhythmic episode with respect to normal sinus rhythm complexes. Symbols representing the arrhythmic complexes are then plotted as a function of the calculated similarity values and the dissimilarity values on an interactive display screen. Additional information relating to the arrhythmic event is elicited through interacting with the symbols displayed on the interactive display screen.

Arias et. al. discuss in "A single implantable cardioverter-defibrillator shock unmasking an electrical storm of 389 ventricular tachycardia episodes triggering device therapies" (American Journal of Emergency Medicine (2008) vol. 26, no.9 pages 1066.e1 - 1066.e3) describe the case of a patient with ischemic cardiomyopathy who presented the first ICD shop approximately 5 months after implantation after occurance of 389 VT episodes terminated by ATP except for the episode requiring shock.

### SUMMARY OF THE INVENTION

Embodiments of the invention are related to medical systems and methods that can be used to communicate with and collect information from implanted medical devices, amongst other things.

In one embodiment, a system for automatically adjudicating arrhythmia episode information includes an episode database having episode data regarding a plurality of different arrhythmia episodes generated from at least one data-generating device and an adjudication processor configured to receive as input episode data regarding one of the different arrhythmia episodes and output characterization data characterizing the input episode data, wherein the characterization data includes an arrhythmia classification. The arrhythmia classification is selected from a group comprising at least monomorphic ventricular tachycardia (MVT). The system further includes an episode processor configured to process the characterization data and episode data, provide at least one report on the characterization data related to a plurality of the different arrhythmia episodes, and provide at least one programming recommendation or at least one alert.

In another embodiment, a system for automatically adjudicating arrhythmia episode information includes at least one cardiac rhythm management device that is implantable in a patient and an episode database having episode data regarding a plurality of different arrhythmia episodes generated from the at least one cardiac rhythm management devices that is implantable in a patient. The system further includes an adjudication processor configured to receive as input episode data regarding one of the different arrhythmia episodes and output characterization data characterizing the input episode data, wherein the characterization data includes an arrhythmia classification. The arrhythmia classification is selected from a group comprising at least polymorphic ventricular tachycardia/ventricular fibrillation (PVT/VF), monomorphic ventricular tachycardia (MVT) and atrial fibrillation (AF). The episode data input includes at least a portion of an intracardiac electrogram. The system further includes an adjudication database for storing the characterization data output by the adjudication processor and an episode processor configured to process characterization data and episode data, provide at least one programming recommendation for the data-generating device, and provide a report for a particular patient on the number of arrhythmia episodes classified as PVT/VF and on the number of episodes classified as MVT.

In yet another embodiment, a method for automatically adjudicating arrhythmia episode information includes the steps of providing an episode database having episode data regarding a plurality of different arrhythmia episodes generated from at least one data-generating device, processing the episode data for one of the different arrhythmia episodes, and outputting characterization data characterizing the episode data. The characterization data includes an arrhythmia classification, wherein arrhythmia classification is selected from a group comprising at least monomorphic ventricular tachycardia (MVT). The method further includes processing the characterization data, providing at least one report on the characterization data, and providing at least one programming recommendation or at least one alert to the data-generating device.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope of the present invention is defined by the appended claims.

### BRIEF DESCRIPTION OF THE DRAWINGS

The invention may be more completely understood in connection with the following drawings, in which:
FIG. 1 is a flowchart illustrating the steps of one embodiment of a method for automated adjudication of arrhythmia episodes.
FIG. 2 is a flowchart illustrating the steps of one embodiment of an overwrite process.
FIG. 3 is a flowchart illustrating the steps of another embodiment of a method for automated adjudication of arrhythmia episodes.
FIG. 4 is a schematic diagram of an exemplary implementation of a cardiac rhythm management (CRM) system, including an implanted CRM device, an external interface device, and a patient management computer system, consistent with at least one embodiment of the invention.
FIG. 5 is a schematic illustration of a patient management system consistent with at least one embodiment of the invention.
FIG. 6 is a schematic diagram of an implementation of the components of an external interface device such as a programmer, in accordance with various embodiments.
FIG. 7 is a schematic view of components of one example of a data-generating device in accordance with an embodiment of the invention.

While the invention is susceptible to various modifications and alternative forms, specifics thereof have been shown by way of example and drawings, and will be described in detail.

### DETAILED DESCRIPTION OF THE INVENTION

This disclosure relates generally to medical data-generating devices and, more particularly, to systems that collect information from medical devices. In particular, this disclosure relates to systems and methods for analyzing and utilizing the information collected. In some embodiments, the data-generating devices are implanted. In some embodiments, the information gathered includes an intracardiac electrogram. In some embodiments, the data-generating devices are devices that monitor cardiac rhythms. Such devices may be a part of a cardiac rhythm management system (CRM systems) that includes an implantable cardiac rhythm management device (CRM device), an external interface device and a patient management computer system.

Some implantable medical devices can be used to provide pacing therapy to patients who have cardiac rhythm problems. For example, an implanted cardiac rhythm management (CRM) device can be used to provide pacing therapy to a patient with sinus node dysfunction, where the heart fails to properly initiate depolarization waves, or an atrio-ventricular conduction disturbance, where the conduction of depolarization waves through the heart tissue is impaired.

Many types of CRM devices communicate with devices located outside of the body, which can receive information from the implanted device including sensor information and information about events, such as when the implanted device has provided therapy. In some cases, the external interface device can also transmit operational parameters to an implanted CRM device, that is, program the device.

These external interface devices can be provided to a patient, often in a patient's home, and can collect information from the implanted device, and provide that information to a computer system designed to monitor the patient's status. An exemplary remote patient management system is the LATITUDE® patient management system, available from Boston Scientific Corporation, Natick, MA. Aspects of exemplary remote patient management and monitoring systems are described in U.S. Pat. No. 6,978,182.

The existence of remote patient management systems such as the LATITUDE® patient management system has provided a large amount of data about patients with implanted medical devices. For example, these systems store information about patient characteristics, patient sensor readings including electrocardiogram (EGM), device settings and delivery of therapy by the device. The sensor readings can include information associated with arrhythmia episodes experienced by the patient.

In one embodiment, a system uses this storehouse of patient-related data to analyze the device performance, understand a particular patient, understand a patient population group or improve therapy provided by the device. Such a system may operate outside of the device itself, such as on a server that is not at the same location as any of the data-gathering devices. As a result, a large amount of computer processing resources and memory can be devoted to utilizing the patient-related data.

"Arrhythmia episode" is defined to mean activity of a patient's body within a time period of particular interest. The time period can be a time when there is abnormal activity, for example, abnormal cardiac activity. "Episode data" is defined to include sensor readings from a medical data-generating device before, during and after the episode, and can also include device settings, actions that were taken by the device and other information. According to the system described herein, an episode database stores episode data about episodes that have occurred.

One or more data-generating devices generate episode data about arrhythmia episodes. The episode database may have episode data about a plurality of arrhythmia episodes generated by one device, or generated by multiple devices. In one embodiment, the episode database is external to any of the data-generating devices. However, in another embodiment, the episode database is located within one of the data generating devices. The episode data can include demographic information about the patient, an electrogram from during the arrhythmia episode, an indication of any therapy that was administered during the episode (such as shock or anti-tachycardia pacing), an indication of the patient's response to any therapy, and information about the patient's condition.

The episode data may also include information from the patient's electronic medical record, device settings at the time of the episode, counter information such as the patient's atrial arrhythmia burden, the patient's drug therapy history, and the patient's ablation history. The patient's ablation history may indicate complete heart block, for example. There are many other examples of possible episode data, which will be further described herein.

The episode data or part of the episode data for a particular episode can be analyzed using an adjudication algorithm to determine an arrhythmia classification and other types of characterization data about the arrhythmia episode. The characterization data can be stored in an adjudication database. In some embodiments, the characterization data is sent to the data-generating device to be stored. Once an arrhythmia classification has been generated for a particular episode or a group of episodes, then it is possible to provide patients and clinicians with many different types of reports related to the episode data. It is also possible for the system to analyze the characterization data to provide programming recommendations for the data-generating device where certain conditions are present. It is also possible to query the adjudication database for many different types of information that may be useful to clinicians, researchers or regulators. Also, the system may be configured to issue alerts for situations that merit attention, where the particular conditions that define those situations are present. One such alert is a patient disposition recommendation, such as an instruction for the patient to go to an emergency room to seek immediate medical help or an instruction for a patient to schedule a clinic visit in the near future.

One example of an arrhythmia episode data-generating device is an implantable cardiac rhythm management arrhythmia episode data-generating device. Specific implantable cardiac rhythm management devices include a pacemaker, a cardioverter-defibrillator, a cardiac resynchronization device, a heart rhythm monitoring device, or the like. However, it is also possible to generate episode data from external devices, including external pacemakers, external cardioverter-defibrillators, and external resynchronization devices. Additional examples of external devices that monitor cardiac activity include ambulatory electrocardiography devices or Holter monitors, which continuously monitor electrical activity of the heart for 24 hours or more. A data-generating device is one that is capable of providing information about an arrhythmia episode experienced by a particular patient.

An adjudication processor is capable of extracting certain features from the episode data which are very helpful in properly classifying the arrhythmia episode. The features may be based on domain knowledge used by physicians to classify the episode data. For example, the electrogram can be used to determine if the arrhythmia episode originates from the atrium or ventricle of the heart through analyzing the timing of the atrial and ventricle activities. The determination can alternatively or in addition be based on the morphology information from the electrograms from different atrial and ventricular channels.

In one embodiment, the adjudication processor is operatively connected to the episode database and is configured to receive as input episode data regarding one of the different arrhythmia episodes. The adjudication processor uses an automated method or algorithm to generate characterization data about the arrhythmia episode. More than one algorithm can be used. For example, different algorithms can be used for different applications. Examples of classification algorithms that can be used for the adjudication algorithm include decision tree algorithms, Naive Bayes algorithms, support vector machine algorithms, and other pattern recognition algorithms.

One example of characterization data is an arrhythmia classification. The characterization data is stored in an adjudication database. In one example, the options for the arrhythmia classification include monomorphic ventricular tachycardia (MVT), polymorphic ventricular tachycardia/ventricular fibrillation (PVT/VF), supraventricular tachycardia (SVT), atrial-ventricular (AV) nodal re-entrant tachycardia (AVNRT), atrial fibrillation (AF), atrial flutter (AFL), atrial tachy (AT), sinus tachy (ST), dual tachy (DT), pacemaker-mediated tachycardia (PMT), one-to-one AV ratio with constant ventricular morphology, oversensing, noise and indeterminate. It is also possible for the system to provide more or fewer arrhythmia classifications than those listed. In one embodiment, the arrhythmia classification options include at least MVT. In one embodiment, the arrhythmia classification options include at least polymorphic ventricular tachycardia/ventricular fibrillation (PVT/VF), monomorphic ventricular tachycardia (MVT) and atrial fibrillation (AF). In various embodiments, there are three or more, four or more, five or more and six or more options for the arrhythmia classification.

In one embodiment, another aspect of the characterization data is an annotation of which part of the episode data was the basis for the arrhythmia episode classification. For example, the system may specify in the characterization data that a portion of the electrogram was the basis for the arrhythmia classification determination, or that multiple portions of, or the entire electrogram was the basis for the arrhythmia classification. For example, the user could note that the atrial, ventricle, shock or a combination of the parts of the electrogram was the basis for the arrhythmia classification. The characterization data can also include a likelihood, or confidence index, associated with each arrhythmia classification. In another embodiment, the annotation may include a justification for the characterization. For example, the episode may be characterized as MVT because a ventricular rate is higher than an atrial rate and only one morphology of the arrhythmia is observed. The characterization data may also include an annotation of a module of a decision process that leads to the arrhythmia episode classification.

Another possible aspect of the characterization data is a classification of the arrhythmia episode as being hemodynamically stable or hemodynamically unstable. A hemodynamically unstable arrhythmia typically cannot be tolerated by patients and needs to be treated immediately. Some SVT or AF arrhythmia episodes may be hemodynamically unstable. For a hemodynamically stable arrhythmia, the therapy may be withheld to allow the arrhythmia episode to resolve on its own. The classification of whether an arrhythmia episode is hemodynamically stable or hemodynamically unstable can be obtained from additional physiological sensors, such as a pressure sensor, and can be applied to most types of arrhythmias. The following published U.S. Patent Applications discuss examples of implementations of hemodynamic stability detection from implantable sensors for tachyarrhythmia classifications: U.S. Published Patent Application Nos. 2007/0142866, 2008/0051843 and 2008/0281367.

An episode processor is provided in one embodiment of the system to perform processing of the adjudication database such as in order to provide reports, patient alerts including patient disposition recommendations, or programming recommendations. A user interface device may also be provided in order to provide these to a user.

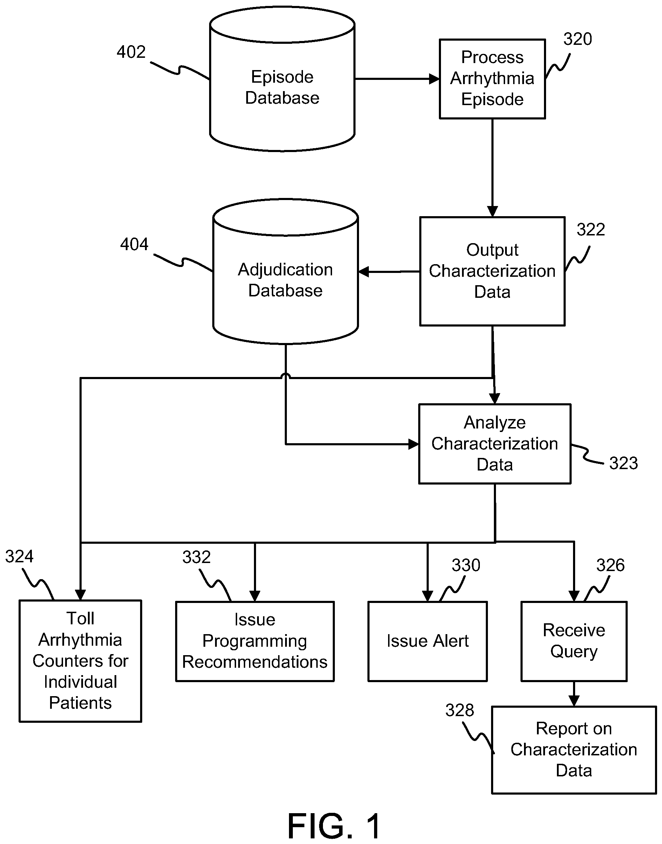

In one embodiment, a system is configured to process a single episode at a time from a data-generating device. In one embodiment, such a system is located in the data-generating device, while in another embodiment the system is separate from the data-generating device and merely receives episode data from the data-generating device. FIG. 1 is a flowchart illustrating one embodiment of how such a system may operate. The episode data relating to the arrhythmia episode is stored in the episode database 402 and is accessed in step 320 as the arrhythmia episode is processed using an arrhythmia adjudication algorithm to provide an output of characterization data in step 322. The characterization data may include an arrhythmia classification, and is stored in the adjudication database 404. Further analysis of the characterization data can be undertaken at step 323, where the adjudication database is accessed to provide programming recommendations, issue alerts or provide reports, as described further herein.

The episode database 402 may contain only a single episode, or the episode database may be a library of arrhythmia episodes from one data-generating device. The episode database may also be a library of arrhythmia episodes from multiple data-generating devices.

The system may be configured to toll an arrhythmia counter for individual patients at step 324 as the characterization data is output to provide a record of the number of different types of arrhythmia episodes that a particular patient has experienced. For example, the episode processor is configured in one embodiment to track and provide a report for a particular patient on the number of arrhythmia episodes classified as polymorphic ventricular tachycardia/ventricular fibrillation (PVT/VF), on the number of arrhythmia episodes classified as supraventricular tachycardia (SVT), and on the number of arrhythmia episodes classified as MVT. The episode processor may further be configured to track and provide a report for a particular patient on the number of arrhythmia episodes classified as one or more of the following: polymorphic ventricular tachycardia/ventricular fibrillation (PVT/VF), supraventricular tachycardia (SVT), AV nodal re-entrant tachycardia (AVNRT), atrial fibrillation (AF), atrial flutter (AFL), atrial tachy, sinus tachy (ST), dual tachy (DT), pacemaker-mediated tachycardia (PMT), one-to-one, oversensing, noise, indeterminate, hemodynamically stable and hemodynamically unstable. The system may also provide counters for any instances when inappropriate therapy was delivered during atrial fibrillation or during SVT.

The system may also categorize the morphology of episodes within a specific arrhythmia classification, such as SVT or MVT. The system may also categorize the response of the patient to therapy. The system may also be configured to provide historical trending for individual patients to show the change in the types, timing and morphology of arrhythmia episodes that a particular patient has experienced over time. The system may receive a query at step 326 and provide a report on the characterization data at step 328. There are many other types of reports that can be provided. The system may be automatically queried to produce the report each time an episode is processed. The system may also generate a report for a pre-set number of queries on a regular basis, such as daily, weekly, or monthly.

Based on analysis of the characterization data at step 323, the system can also be configured to issue an alert at step 330 if a condition exists that merits attention. For example, an alert can be issued if ventricular anti-tachycardia therapy was delivered to a patient during an arrhythmia episode classified as SVT, AF or atrial flutter (AFL). An alert can be issued if a shock was delivered without any anti-tachycardia pacing (ATP) being attempted before the shock during an arrhythmia episode classified as MVT. If an episode is classified as oversensing, an alert may be issued. Another scenario where an alert is desirable is where a ventricular tachycardia that required treatment, such as a hemodynamic unstable SVT, was not detected by a cardiac rhythm management device, and so no treatment was provided during that episode. Yet another scenario where an alert is desirable is where a shock was delivered to a hemodynamically stable arrhythmia.

Another type of alert that may be issued is a patient disposition recommendation, which is a communication directed to the patient or a caregiver to take a specific action, which can be delivered through a patient management system. For example, if multiple arrhythmias are observed in a very short duration of time, the recommendation may be, "Seek immediate medical help." If the arrhythmia is similar to arrhythmia episodes observed before, and the device behaved properly, the recommendation may be, "Device functioning properly. Nurse, please call to check on patient." If the arrhythmia is different from what were observed before, or programming changes are needed, the recommendation may be, "Schedule a clinic visit." The particular patient disposition recommendations and when the recommendations are provided may be selected by a caregiver such as a physician.

The system can also be configured to provide programming recommendations at step 332 based on the analysis of the episode data and the characterization data. The system may recommend changes to the detection parameters of the data-generating device, such as adjustment of the parameters related to tachycardia detection, including the number of tachy zones, the rate cutoff, the initial detection durations, sustained rate duration (SRD), and other parameters. For example, if MVT is observed in the VF zone, then the system may recommend that the VF zone threshold be increased.

Other tachycardia detection parameters that may be suggested to be modified include morphology-based discrimination features and turning on or off specific or all detection enhancements. Other possible recommendations for detection parameters include reprogramming adjustable threshold values, for example, the atrial fibrillation rate and a stability threshold.

The system may also recommend programming changes that govern how therapy is delivered. For example, tachycardia therapy parameters may be changed to benefit the patient. For example, if MVT is observed and anti-tachycardia pacing (ATP) is turned off, the system may recommend turning on the anti-tachycardia pacing. In another example, use of ATP may be recommended where the arrhythmia classification is MVT, where MVT was previously treated with shock, and where ATP had not previously been tried for a patient. In yet another example, if ATP is not effective to assist the patient during a particular arrhythmia episode, the system may recommend switching the therapy settings to a different setting. The system may also recommend changing brady/sensing parameters, such as sensitivity and refractory parameters when oversensing/undersensing is detected.

Another programming recommendation that can be made is to change the parameters for storage of data in the data generating device. For example, if an arrhythmia classification is indeterminate, the system may recommend that a five-minute data interval be retained before each episode for future episodes. In another example, if the arrhythmia classification is one-to-one AV ratio with constant ventricular morphology, the system may recommend a detection parameter enhancement to look for gradual onset of tachycardia, which would indicate sinus tachycardia. If the detection enhancement revealed sudden atrial onset, then atrial tachycardia would be indicated.

Alerts, reports and programming recommendations can be graphically displayed for a patient, patient caregiver or other user of the system. This display can be provided via external interface devices or other user interface devices associated with the system, as further described herein. In one embodiment, the programming recommendations are presented to a patient caregiver for approval and implementation. In another embodiment, the programming recommendations or certain programming recommendations are automatically implemented in a data-generating device, and the patient caregiver is notified of the changes.

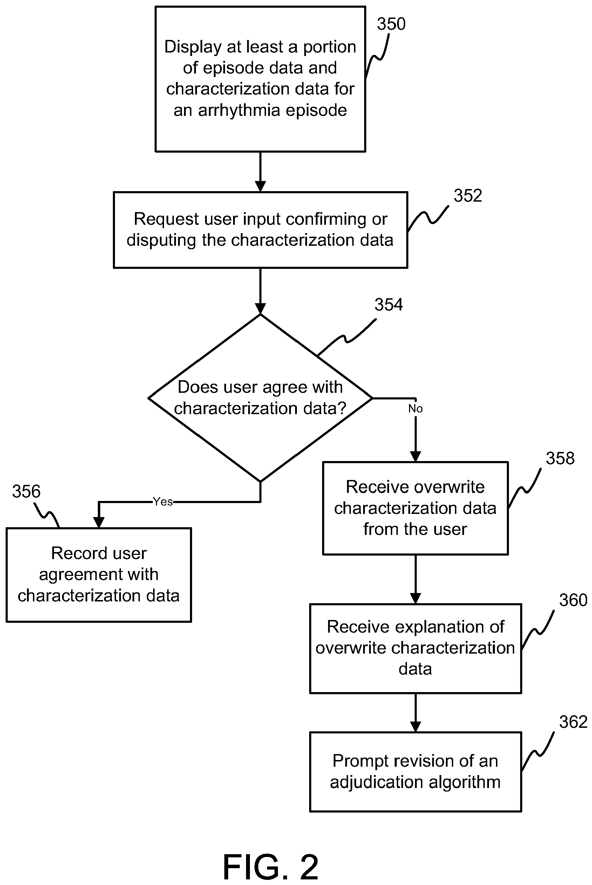

In one embodiment, a user of an automated adjudication system is given an opportunity to review the arrhythmia classification and other characterization data determined by the adjudication processor for a particular arrhythmia episode and overwrite that information. FIG. 2 is a flowchart illustrating the steps of one embodiment of an overwrite process. First, at least a portion of the episode data is displayed for the user in step 350 along with at least a portion of the characterization data for a particular arrhythmia episode. Then, user input is requested at step 352 either confirming or disputing the characterization data. If the user agrees with the characterization data at step 354, then the user's agreement is recorded in the characterization database at step 356. If the user does not agree with the characterization data, then the system receives overwrite characterization data from the user at step 358. In one embodiment, the system also receives an explanation of the overwrite data at step 360 from the user. Such an explanation may identify part or all of the electrogram as being the basis for the overwrite data. The overwrite data may include a different arrhythmia classification, changes to other aspects of the characterization data, or changes to multiple aspects of the characterization data. In at least one embodiment, the system is configured to reclassify the arrhythmia classification of the arrhythmia episode based on the overwrite characterization data in step 358.

Many different user interfaces may be used to permit overwriting of the data by displaying information for the user and accepting input from the user. A user interface device connected to a patient monitoring system can be used to overwrite the data.

Particular arrhythmia episodes where an overwrite operation is carried out represent situations for which it may be possible for the arrhythmia adjudication algorithm to be fine-tuned and improved. For example, the adjudication algorithm can be fine-tuned for an individual patient on the basis of the overwritten results. An individualized algorithm may be developed for a particular patient. An adjudication algorithm may also be catered to a particular set of patient characteristics. In addition, an adjudication algorithm applied broadly can also be improved and fine-tuned on the basis of overwritten results. Over time, an adaptive adjudication algorithm can be developed based on user input. The system prompts the user to consider revising the adjudication algorithm at step 362. The modified algorithm may be applied to the previous episodes from the patient or other patients to re-characterize the previous episodes.

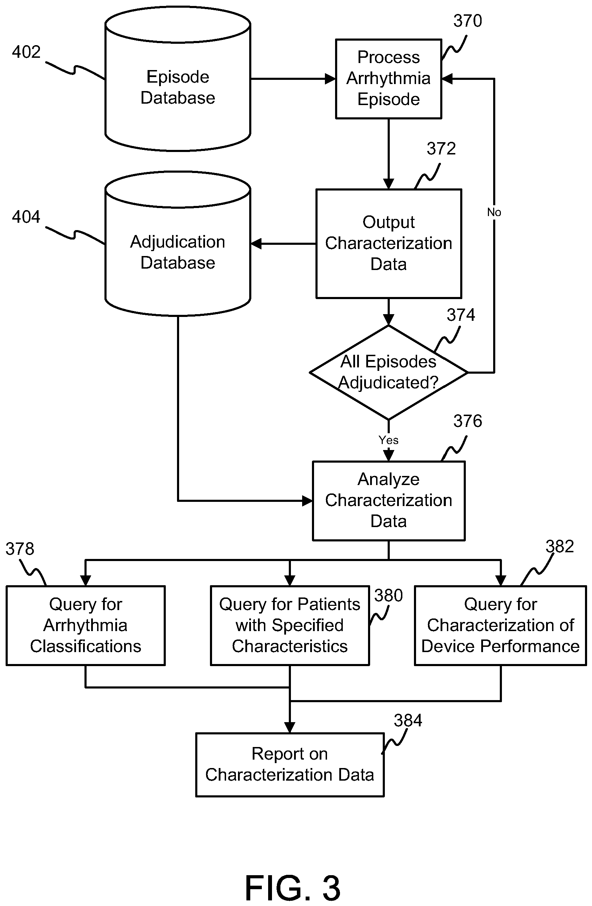

Where an episode database contains arrhythmia episode data from many different patients, it is possible to perform many different types of analysis using that data to analyze device performance, improve device operation, analyze different patient groups, analyze different arrhythmia types or analyze arrhythmias from a particular clinic. FIG. 3 is a flowchart of one embodiment of a method for performing analysis on an episode database containing episode data from many different patients and devices. Arrhythmia episodes in the episode database 402 are processed at step 370 to provide characterization data at step 372, which is stored in the adjudication database 404. The system checks for whether all episodes have been adjudicated at step 374, and continues to process arrhythmia episodes until all have been adjudicated.

Analysis of the characterization data at step 376 can provide many different types of reports, including the reports previously described. Queries may be performed on types of arrhythmias at step 378. One possible query specifies the arrhythmia type as determined by the adjudication algorithm in prior adjudications. The system may provide a report on the percentages of episodes from a particular patient, such as the percentage of episodes classified as VF, AF, noise, MVT and other. Another possible query is the percentages of arrhythmia types in the entire library.

Queries may also be performed for patients or for episodes experienced by patients in specific populations groups or with specific characteristics at step 380. For example, the system may be queried for patients with specified characteristics, such as a history of AF episodes or an increase of X% in the AF burden within the last Y months. The system may also report on the episodes in a particular patient segment, such as gender group, age group, race group, a group with specific medical history factors, a group of patients at a particular clinic, a group of patients within a particular geographic region, or within a particular country.

It is also possible to query the system to obtain reports on device performance and therapy delivery at step 382. Examples of therapy that can be provided by a CRM device include ventricular anti-tachycardia therapy such as ventricular anti-tachycardia pacing, cardioversion and shock. A report on therapy delivery can be broken down into categories such as non-treated and/or non-sustained episodes, anti-tachycardia pacing (ATP) only, and shock therapy.

A query may combine one or more of patient, episode and therapy characteristics. Each of the queries discussed herein and many more can be used to generate a report at step 384.

Further examples of episode data will now be described. In one embodiment, the episode data can include a statement of the device type or device types which generated the data, such as a Holter monitor, pacemaker, single chamber implantable cardioverter defibrillator (ICD), dual chamber ICD, cardiac resynchronization therapy defibrillator (CRT-D) or other devices. Some devices generate an arrhythmia classification, such as non-sustained ventricular tachy arrhythmia (VT), sustained ventricular tachy arrhythmia, atrial fibrillation (AF) and supraventricular tachycardia (SVT). This device-generated arrhythmia classification may also be part of the episode data.

It is also possible that the episode data includes caregiver annotations related to the particular arrhythmia episode. Caregiver annotations may include an assigned arrhythmia classification and many other types of information related to the particular episode.

The episode data can also include electrocardiogram (EGM) recorded before, during and after the duration of the episode. The EGM may be generated by an implanted device. The EGM is an intracardiac EGM in one embodiment.

In one embodiment, the episode data includes both a first and second EGM. The first EGM may be an intracardiac EGM from an implanted device, while the second EGM is a surface EGM from an external device. In one embodiment, both a first and second EGM within the episode data are an intracardiac EGM.

The episode data may also include sensor readings from the patient including physiologic sensors such as a subcutaneous electrogram sensor, pressure sensor, an accelerometer, a temperature sensor and an impedance sensor. Other types of sensor readings that can be included in episode data include readings from a sleep sensor, functional capacity indicator, autonomic tone indicator, sleep quality indicator, cough indicator, anxiety indicator, and cardiovascular wellness indicator for calculating a quality of life indicator quantifying a patient's overall health and well-being. Additional sensor readings that can be included are from a thermometer, a sphygmomanometer or other external devices.

In one embodiment, the episode data also includes pace and sense markers on the EGM, which can indicate when along the course of the EGM the patient's heart rate can be classified in certain ways, such as ventricular tachy arrhythmia (VT), atrial fibrillation (AF) and supraventricular tachycardia (SVT).

In one embodiment, the episode data also includes device diagnostics which are measurements recorded by the device, and which can include average atrial heart rate, average ventricular heart rate and many other measurements.

The episode data can also include the devices response to the arrhythmia, such as anti-tachycardia pacing (ATP) therapy or shock therapy, and more specific descriptions of the therapy. The response information can be shown on the EGM strip and/or textually. In certain embodiments, the episode data can further include information on how the patient responded to the therapy, and information on subsequent therapy that was delivered.

In addition, the episode data can also include the device settings that were present during the episode. Examples of device settings include therapy settings such as what type of pacing or shock therapy will be applied when the device concludes that certain cardiac arrhythmias are occurring. For example, ATP therapy involves delivering a series of timed pacing pulses where it is possible to define the number of bursts delivered, the number of pacing pulses within each burst, and many other criteria. ATP therapy can fall into several different schemes, and each device has its own terminology describing the ATP therapy schemes. For some devices available from Boston Scientific Corporation, some of the ATP therapy schemes are named a burst scheme, a ramp scheme, and scan scheme or a ramp/scan scheme. The specifics of these ATP therapy scheme names, and when they will be delivered to the patient, are examples of device settings that may be a part of the episode data.

Another type of device setting is shock therapy parameters, which govern the shock energy, timing of shock delivery, shock vector configuration, as well as many other qualities of shock therapy.

Other types of device setting include detection settings that set forth the heart rate, rate of onset, and other criteria by which the device will classify the episode as certain arrhythmia types. Another type of device setting is frequently referred to as detection enhancements. These settings can be used to ensure that it is appropriate to deliver therapy to the patient by checking for the presence or absence of certain criteria. If the criteria are present, the detection enhancement settings may cause the device to delay or inhibit therapy delivery, bypass therapy inhibition, or bypass a sequence of ATP therapy in favor of shock therapy. Examples of specific detection enhancement criteria include the following, where the name for the programming feature for some devices available from Boston Scientific Corporation is included in parenthesis: certain therapy inhibitor conditions can be bypassed if the ventricular rate is greater than the atrial rate (V Rate > A Rate), ventricular therapy can be inhibited if the atrial rhythm is too fast (AFib Rate Threshold), ventricular therapy can be inhibited if the ventricular rhythm is unstable (Stability), and ventricular therapy can be inhibited if the patient's heart rate increases gradually (Onset). These parameters and whether they are turned on or off can be displayed along with other device settings in one embodiment.

Another aspect of the episode data is physiologic data collected by sensors such as a pressure sensor, an accelerometer, a temperature sensor or an impedance sensor. The episode data may also include physiologic data from many other types of sensors.

Further detailed embodiments of the hardware of the system will now be described with respect to the attached FIGS.

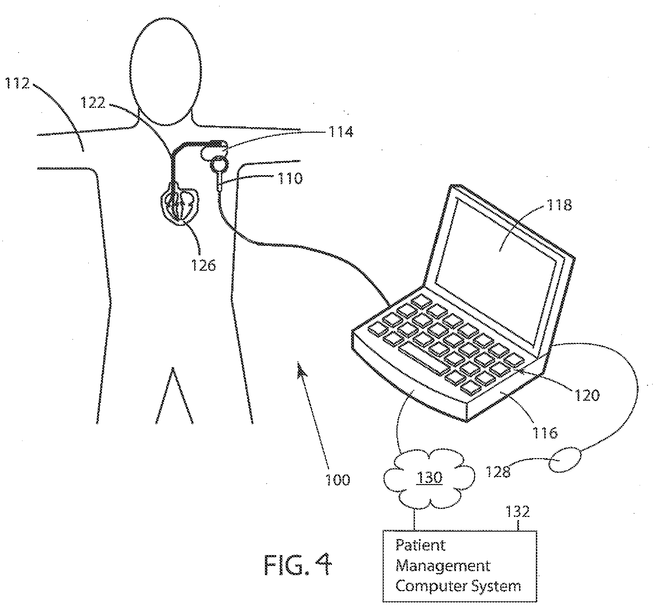

One embodiment of a data-generating device is a CRM device, as will now be described with reference to FIG. 4, which is a schematic of an exemplary CRM system 100. The system 100 can include an implantable medical device 114 disposed within a patient 112. The implantable medical device 114 can include pacing functionality. The implantable medical device 114 can be of various types such as, for example, a pacemaker, a cardioverter-defibrillator, a cardiac resynchronization device, a heart rhythm monitoring device, or the like. In some embodiments, the implantable medical device 114 can include one or more leads 122 disposed in or near the patient's heart 126.

The implantable medical device 114 can be in communication with an external interface system 116. In some embodiments, communication between the implantable medical device 114 and the external interface system 116 can be via inductive communication through a wand 110 held on the outside of the patient 112 near the implantable medical device 114. However, in other embodiments, communication can be carried out via radiofrequency transmission, acoustically, or the like.

The implantable medical device 114 can include one or more implantable sensors in order to gather data regarding the patient 112. For example, the implantable medical device 114 can include an activity level sensor, a respiration sensor, a blood pressure sensor, an impedance sensor, or other sensors.

The implantable medical device 114 can be configured to store data over a period of time, and periodically communicate with the external interface system 116 in order to transmit some or all of the stored data.

The external interface system 116 can be for example, a programmer, a programmer/recorder/monitor device, a computer, a patient management system, a personal digital assistant (PDA), or the like. As used herein, the term programmer refers to a device that programs implanted devices, records data from implanted devices, and allows monitoring of the implanted device. Exemplary programmer/recorder/monitor devices include the Model 3120 Programmer, available from Boston Scientific Corporation, Natick, MA. The external interface system 116 can include a user input device, such as a keyboard 120 and/or a mouse 128. The external interface system 116 can include a video output channel and video output device, such as a video display 118 for displaying video output. The displayed video output can include a user interface screen. In addition, the video display 118 can also be equipped with a touch screen, making it into a user input device as well.

The external interface device 116 can display real-time data and/or stored data graphically, such as in charts or graphs, and textually through the user interface screen. In addition, the external interface device 116 can present textual information to a user along with several response options. The external interface device 116 can also input and store a user's response to a question, and can store a user's text response in some embodiments.

In one embodiment, the external interface device 116, which can also be referred to as a user interface, is in communication with a patient management computer system 132. The communication link between the user interface 116 and the patient management computer system 132 may be via phone lines, the Internet 130, or any other data connection. The user interface 116 can also be used when it is not in communication with a device, but is only in communication with the patient management computer system 132.

In one embodiment, the external interface device 116 is capable of changing the operational parameters of the implantable medical device 114, and is therefore referred to as a programmer. Typically, programmers are used to interface with CRM devices in a clinic or hospital setting. In this context, the user of the external interface device is a physician or trained technician.

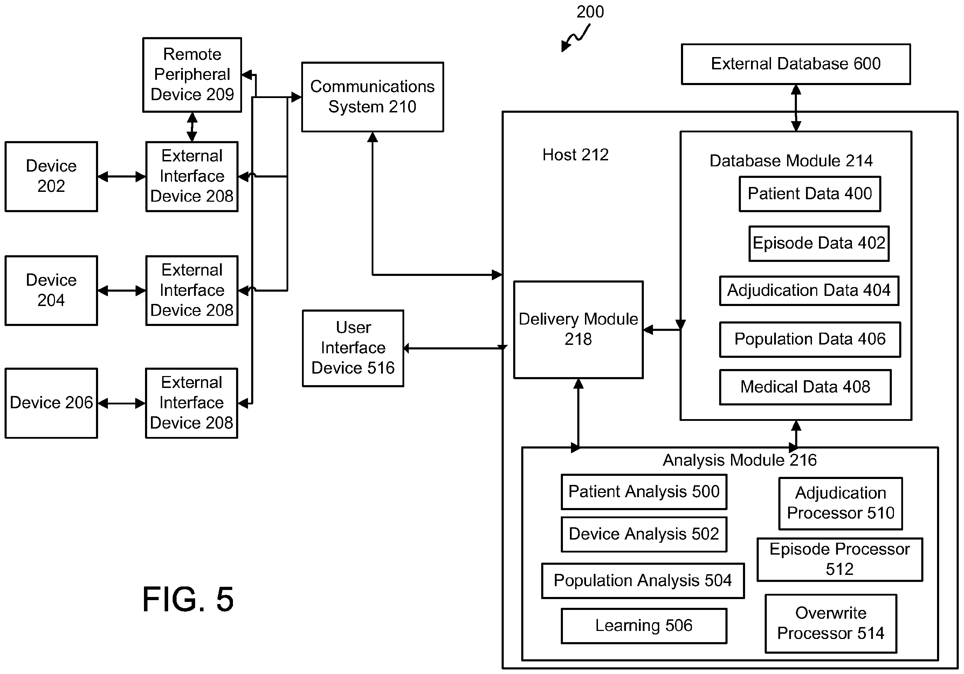

FIG. 5 is a schematic illustration of a patient management system consistent with at least one embodiment of the invention. The patient management system is capable of generating an episode database and supporting a training module. Patient management system 200 generally includes one or more devices 202, 204, and 206, one or more external interface devices 208, a communication system 210, one or more remote peripheral devices 209, and a host 212.

Each component of the patient management system 200 can communicate using the communication system 210. Some components may also communicate directly with one another. The various components of the example patient management system 200 illustrated herein are described below.

Data-generating devices 202, 204, and 206 can be implantable devices or external devices that may provide one or more of the following functions with respect to a patient: (1) sensing, (2) data analysis, and (3) therapy. For example, in one embodiment, devices 202, 204, and 206 are either implanted or external devices used to measure a variety of physiological, subjective, and environmental conditions of a patient using electrical, mechanical, and/or chemical means. The devices 202, 204, and 206 can be configured to automatically gather data or can require manual intervention by the patient or another person. The devices 202, 204, and 206 can be configured to store data related to the physiological and/or subjective measurements and/or transmit the data to the communication system 210 using a variety of methods, described in detail below. Although three devices 202, 204, and 206 are illustrated in the example embodiment shown, many more devices can be coupled to the patient management system. In one embodiment, each of the devices 202, 204 and 206 is serving a different patient. In one embodiment, two or more devices are serving a single patient.

The devices 202, 204, and 206 can be configured to analyze the measured data and act upon the analyzed data. For example, the devices 202, 204, and 206 can be configured to modify therapy or provide an alarm based on the analysis of the data.

In one embodiment, devices 202, 204, and 206 provide therapy. Therapy can be provided automatically or in response to an external communication. Devices 202, 204, and 206 are programmable in that the characteristics of their sensing, therapy (e.g., duration and interval), or communication can be altered by communication between the devices 202, 204, and 206 and other components of the patient management system 200. Devices 202, 204, and 206 can also perform self-checks or be interrogated by the communication system 210 to verify that the devices are functioning properly. Examples of different embodiments of the devices 202, 204, and 206 are provided herein.

Devices implanted within the body have the ability to sense and communicate as well as to provide therapy. Implantable devices can provide direct measurement of characteristics of the body, including, without limitation, electrical cardiac activity (e.g., a pacemaker, cardiac resynchronization management device, defibrillator, etc.), physical motion, temperature, heart rate, activity, blood pressure, breathing patterns, ejection fractions, blood viscosity, blood chemistry, blood glucose levels, and other patient-specific clinical physiological parameters, while minimizing the need for patient compliance. Derived measurements can also be determined from the implantable device sensors (e.g., a sleep sensor, functional capacity indicator, autonomic tone indicator, sleep quality indicator, cough indicator, anxiety indicator, and cardiovascular wellness indicator for calculating a quality of life indicator quantifying a patient's overall health and well-being).

Devices 202, 204, and 206 can also be external devices, or devices that are not implanted in the human body, that are used to measure physiological data (e.g., a thermometer, sphygmomanometer, or external devices used to measure blood characteristics, body weight, physical strength, mental acuity, diet, heart characteristics, and relative geographic position).

The patient management system 200 may also include one or more remote peripheral devices 209 (e.g., cellular telephones, pagers, PDA devices, facsimiles, remote computers, printers, video and/or audio devices) that use wired or wireless technologies to communicate with the communication system 210 and/or the host 212.

The example database module 214 includes a patient database 400, an episode database 402, an adjudication database 404, a population database 406, and a medical database 408, all of which are described further below. The patient database 400 includes patient specific data, including data acquired by the devices 202, 204, and 206, as well as a patient's medical records and historical information. The population database 406 includes non-patient specific data, such as data relating to other patients and population trends. The example medical database 408 includes clinical data relating to the treatment of diseases, such as historical trend data for multiple patients in the form of a record of progression of their disease(s) along with markers of key events.

The episode database 402 has episode data regarding a plurality of different arrhythmia episodes generated from those of devices 202, 204, and 206 that provide arrhythmia episode data. The adjudication database 404 includes adjudication conclusions associated with the episode data such as arrhythmia episodes. The adjudication database 404 and the episode database 402 can actually be a single database with shared data that is used as either episode data or adjudication data depending on the particular data set being presented to the user.

Information can also be provided from an external source, such as external database 600. For example, the external database 600 includes external medical records maintained by a third party, such as drug prescription records maintained by a pharmacy, providing information regarding the type of drugs that have been prescribed for a patient or, in another example, authorization data from patient groups that have authorized users to view arrhythmia episode data.

The example analysis module 216 includes a patient analysis module 500, device analysis module 502, population analysis module 504, and a learning module 506. Patient analysis module 500 may utilize information collected by the patient management system 200, as well as information for other relevant sources, to analyze data related to a patient and provide timely and predictive assessments of the patient's well-being. Device analysis module 502 analyzes data from the devices 202, 204, and 206 and external interface devices 208 to predict and determine device issues or failures. Population analysis module 504 uses the data collected in the database module 214 to manage the health of a population. Learning module 506 analyzes the data provided from the various information sources, including the data collected by the patient system 200 and external information sources, and may be implemented via a neural network (or equivalent) system to perform, for example, probabilistic calculations. In one embodiment, the learning module uses artificial intelligence to improve its ability to adjudicate arrhythmias using physician-selected diagnoses.

The analysis module 216 further includes an adjudication processor 510, and episode processor 512 and an overwrite processor 514. In one embodiment, the adjudication processor is operatively connected to at least the episode database 402 and is configured to receive as input episode data regarding one of the different arrhythmia episodes. The adjudication processor uses an automated method or algorithm to generate characterization data about the arrhythmia episode. The characterization data, including an arrhythmia classification for each arrhythmia episode that is analyzed, is stored in the adjudication database 404.

The episode processor 512 performs processing of the adjudication database such as in order to provide reports, patient alerts, or programming recommendations. The overwrite processor 514 can analyze data provided from the episode database 402, the adjudication database 404, and other portions of the patient management system 200 to determine what particular portion of episode data for one of the arrhythmia episodes from the episode database should be displayed to a user. Overwrite processor 514 can, through the delivery module 218 described below, provide the means for graphically displaying a portion of data selected from arrhythmia episode data related to an arrhythmia episode of a patient, such as data generated by a data-generating device and stored in the episode database.

Overwrite processor 514 also requests from a user any changes in the characterization data determined by the adjudication processor, and can articulate the request for user input characterizing an arrhythmia episode. The request may be a direct question to a user, a series of choices provided to the user, or simply a blank space on the user interface configured to accommodate the user input. The overwrite processor 514 may also store the overwrite history for individual users.

One or more portions of the analysis module 216, such as the adjudication processor 510 and episode processor 512, may be located remotely from other parts of the patient management system 200.

Delivery module 218 coordinates the delivery of reports, patient alerts or programming recommendations based on the analysis performed by the host 212. For example, based on the data collected from the devices and analyzed by the host 212, the delivery module 218 can deliver information to the caregiver, user, or to the patient using, for example, a display provided on the external interface device 208. A user interface device 516 that is independent of a data-generating device may also be used to deliver information. The external interface device 208 and user interface device 516 are also configured, according to multiple embodiments, to display a report, alert, or programming recommendation, receive overwrite information from a user, and receive other data from the user. Displayed data, as described above, can be determined by the episode processor 512, overwrite processor 514 and delivery module 218.

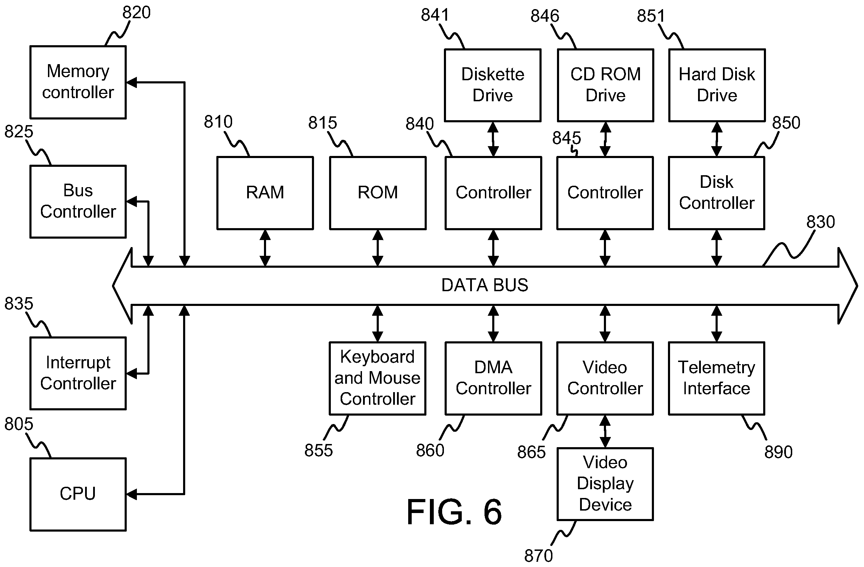

External interface devices 208 to display information, such as programmer/recorder/monitors, can include components common to many computing devices. User interface devices 516 to display and received information from users can also include components common to many computing devices. Referring now to FIG. 6, a diagram of various components is shown in accordance with some embodiments of the invention. However, it is not required that an external interface device have all of the components illustrated in FIG. 6.

In one embodiment, the external interface device includes a central processing unit (CPU) 805 or processor, which may include a conventional microprocessor, random access memory (RAM) 810 for temporary storage of information, and read only memory (ROM) 815 for permanent storage of information. A memory controller 820 is provided for controlling system RAM 810. A bus controller 825 is provided for controlling data bus 830, and an interrupt controller 835 is used for receiving and processing various interrupt signals from the other system components.

Mass storage can be provided by diskette drive 841, which is connected to bus 830 by controller 840, CD-ROM drive 846, which is connected to bus 830 by controller 845, and hard disk drive 851, which is connected to bus 830 by controller 850. User input to the programmer system may be provided by a number of devices. For example, a keyboard and mouse can connected to bus 830 by keyboard and mouse controller 855. DMA controller 860 is provided for performing direct memory access to system RAM 810. A visual display is generated by a video controller 865 or video output, which controls video display 870. The external system can also include a telemetry interface 890 or telemetry circuit which allows the external system to interface and exchange data with an implantable medical device. It will be appreciated that some embodiments may lack various elements illustrated in FIG. 6.

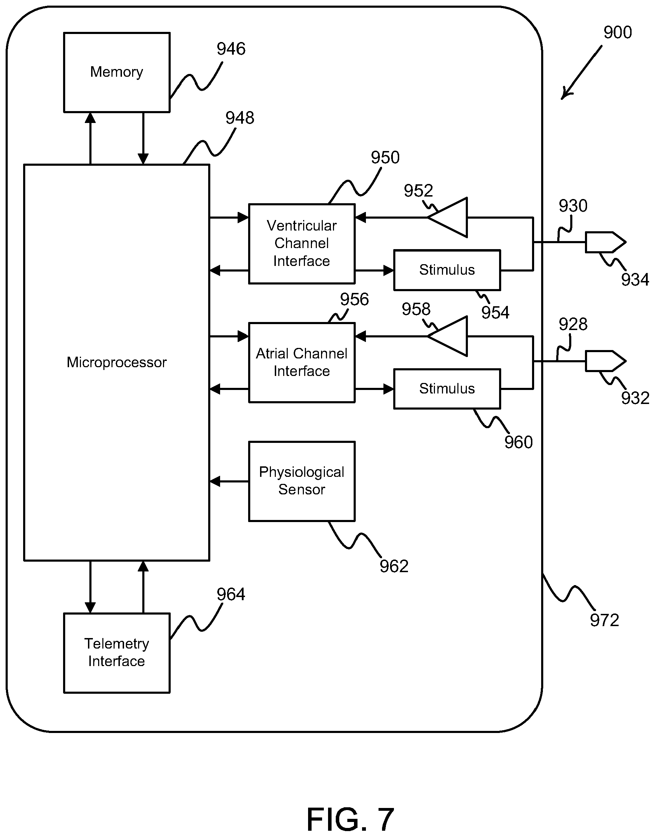

Referring now to FIG. 7, some components of an exemplary implantable system 900 are schematically illustrated. The implantable medical system 900 can include an implantable medical device 972 coupled to one or more stimulation leads 930 and 928. The implantable device 972 can also include one or more physiological sensors 962, or other sensors, such as a pressure sensor, impedance sensor and others.

The implantable device can include a microprocessor 948 (or processor) that communicates with a memory 946 via a bidirectional data bus. The memory 946 typically comprises ROM or RAM for program storage and RAM for data storage. The implantable device can be configured to execute various operations such as processing of signals and execution of methods as described herein. A telemetry interface 964 is also provided for communicating with an external unit, such as a programmer device or a patient management system.

The implantable device can include ventricular sensing and pacing channels comprising sensing amplifier 952, output circuit 954, and a ventricular channel interface 950 which communicates bidirectionally with a port of microprocessor 948. The ventricular sensing and pacing channel can be in communication with stimulation lead 930 and electrode 934. The implantable device can include atrial sensing and pacing channels comprising sensing amplifier 958, output circuit 960, and an atrial channel interface 956 which communicates bidirectionally with a port of microprocessor 948. The atrial sensing and pacing channel can be in communication with stimulation lead 928 and electrode 932. For each channel, the same lead and electrode can be used for both sensing and pacing. The channel interfaces 950 and 956 can include analog-to-digital converters for digitizing sensing signal inputs from the sensing amplifiers and registers which can be written to by the microprocessor in order to output pacing pulses, change the pacing pulse amplitude, and adjust the gain and threshold values for the sensing amplifiers.

It should be noted that, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as "arranged", "arranged and configured", "constructed and arranged", "constructed", "manufactured and arranged", and the like.

One of ordinary skill in the art will understand that the modules, circuitry, and methods shown and described herein with regard to various embodiments of the invention can be implemented using software, hardware, and combinations of software and hardware. As such, the illustrated and/or described modules and circuitry are intended to encompass software implementations, hardware implementations, and software and hardware implementations.

All publications and patent applications in this specification are indicative of the level of ordinary skill in the art to which this invention pertains.

This application is intended to cover adaptations or variations of the present subject matter. It is to be understood that the above description is intended to be illustrative, and not restrictive. The scope of the present subject matter should be determined with reference to the appended claims.

## Claims

1. A system for automatically adjudicating arrhythmia episode information, comprising:
at least one data-generating device, wherein the data-generating device is a cardiac rhythm management device that is implantable in a patient;
an episode database having episode data regarding a plurality of different arrhythmia episodes generated from the at least one data-generating device, wherein the episode data includes at least a portion of an intracardiac electrogram and output from a physiological sensor configuration including one or more sensors selected from the group consisting of a pressure sensor, an accelerometer, a temperature sensor and an impedance sensor;
an adjudication processor configured to receive as input episode data regarding one of the different arrhythmia episodes and output characterization data characterizing the input episode data, wherein the characterization data includes an arrhythmia classification, wherein the arrhythmia classification is selected from a group comprising at least monomorphic ventricular tachycardia (MVT);
an episode processor configured to process the characterization data and episode data, provide at least one report on the characterization data related to a plurality of the different arrhythmia episodes, and to analyze the characterization data to provide programming recommendations for the data-generating device including a recommendation to change the storage parameters for a length of time that data is stored before the detection of a tachy event.

2. The system of claim 1 wherein the episode data further includes one selected from the group consisting of device-generated event markers on the intracardiac electrogram, device diagnostics, therapy delivered during the arrhythmia episode, a portion of a second electrogram, data from an electronic medical record, or data from a manual entry of information into the episode database.

3. The system of claim 1, wherein the system includes an adjudication database for storing the characterization data output by the adjudication processor.

4. The system of claim 1 wherein the programming recommendation comprises one selected from the group of:
(a) a recommendation to use anti-tachycardia pacing (ATP) where the arrhythmia classification is MVT, where MVT was previously treated with shock, and where ATP had not previously been tried for a patient;
(b) a recommendation to switch to a different tachy therapy mode or configuration if an issued therapy was not effective; and
(c) a recommendation to change brady pacing parameters; and
(d) a recommendation to change detection parameters of the data-generating device, wherein the programming recommendation is chosen from the group of turning morphology-based discrimination on or off, and changing electrogram sensing parameters.

5. The system of claim 1 wherein the episode processor is configured to issue an alert if at least one of the events occurs in the group of:
(a) a ventricular antitachycardia therapy was delivered during an arrhythmia episode with an arrhythmia classification of supraventricular tachycardia (SVT),
(b) a ventricular antitachycardia therapy was delivered during an arrhythmia episode with an arrhythmia classification of atrial fibrillation (AF) or atrial flutter (AFL),
(c) a ventricular antitachycardia therapy was delivered due to oversensing,
(d) a shock was delivered without an anti-tachycardia pacing attempt during an arrhythmia episode with an arrhythmia classification of monomorphic ventricular tachycardia (MVT), and
(e) a shock was delivered to a hemodynamically stable arrhythmia episode.

6. The system of claim 1 wherein the episode processor is configured to issue an alert that provides instructions to a patient.

7. The system of claim 1 wherein the episode processor is configured to provide a report for a particular patient on the number of arrhythmia episodes classified as polymorphic ventricular tachycardia/ventricular fibrillation (PVT/VF) and on the number of arrhythmia episodes classified as MVT.

8. The system of claim 1 wherein the episode processor is configured to re-program the data-generating device to execute the programming recommendation.

9. The system of claim 1 wherein the arrhythmia classification is selected from the group further comprising at least polymorphic ventricular tachycardia/ventricular fibrillation (PVT/VF), supraventricular tachycardia (SVT), AV nodal re-entrant tachycardia (AVNRT), atrial fibrillation (AF), atrial flutter (AFL), atrial tachy, sinus tachy (ST), dual tachy (DT), pacemaker-mediated tachycardia (PMT), one-to-one, oversensing, noise, and indeterminate.

10. The system of claim 1 wherein the characterization data includes one selected from the group of:
(a) an annotation of which part of the episode data was the basis for the arrhythmia episode classification;
(b) an annotation of a justification for the characterization;
(c) an annotation of a module of a decision process that leads to the arrhythmia episode classification; and
(d) a classification of the arrhythmia episode as being hemodynamically stable or hemodynamically unstable.

11. The system of claim 1 further comprising a review processor, including a user interface configuredto:
display at least a particular portion of episode data for one of the arrhythmia episodes from the episode database and display the characterization data output by the adjudication algorithm process; and
receive overwrite characterization data from the user characterizing the displayed episode data.

12. The system of claim 11 wherein the system is configured to revise the arrhythmia classification of the arrhythmia episode based on the overwrite characterization data.

13. The system of claim 11 wherein the system is configured to prompt revision of an adjudication algorithm after receiving overwrite characterization data.

14. A method for automatically adjudicating arrhythmia episode information using a medical device system, comprising the steps of:
providing an episode database having episode data regarding a plurality of different arrhythmia episodes generated from at least one data-generating device of the medical device system, wherein the data-generating device is a cardiac rhythm management device that is implantable in a patient, wherein the episode data includes at least a portion of an intracardiac electrogram and output from a physiological sensor configuration including one or more sensors selected from the group consisting of a pressure sensor, an accelerometer, a temperature sensor and an impedance sensor;
processing the episode data for one of the different arrhythmia episodes and outputting characterization data characterizing the episode data wherein the characterization data includes an arrhythmia classification, wherein arrhythmia classification is selected from a group comprising at least monomorphic ventricular tachycardia (MVT); and
processing the characterization data, providing at least one report on the characterization data, and providing programming recommendations for the data-generating device including a recommendation to change the storage parameters for a length of time that data is stored before the detection of a tachy event.

## Patentansprüche

1. System zum automatischen Entscheiden über Arrhythmie-Episodeninformationen, welches aufweist:
zumindest eine Datenerzeugungsvorrichtung, wobei die Datenerzeugungsvorrichtung eine Herzrhythmus-Managementvorrichtung ist, die in einen Patienten implantierbar ist;
eine Episodendatenbank mit Episodendaten betreffend mehrere verschiedene Arrhythmie-Episoden, die von der zumindest einen Datenerzeugungsvorrichtung erzeugt wurden, wobei die Episodendaten zumindest einen Teil eines Elektrointrakardiogramms und die Ausgabe von einer physiologischen Sensorkonfiguration enthaltend einen oder mehrere Sensoren, die ausgewählt sind aus der Gruppe bestehend aus einem Drucksensor, einem Beschleunigungsmesser, einem Temperatursensor und einem Impedanzsensor ausgewählt sind, enthalten;
einen Entscheidungsprozessor, der konfiguriert ist zum Empfangen von Episodendaten betreffend eine der verschiedenen Arrhythmie-Episoden als Eingabe und zum Ausgeben von Charakterisierungsdaten, die die eingegebenen Episodendaten charakterisieren, wobei die Charakterisierungsdaten eine Arrhythmie-Klassifizierung enthalten, wobei die Arrhythmie-Klassifizierung ausgewählt ist aus einer Gruppe aufweisend zumindest monomorphe ventrikuläre Tachykardie (MVT);
einen Episodenprozesser, der konfiguriert ist zum Verarbeiten der Charakterisierungsdaten und der Episodendaten, bereitstellen zumindest eines Berichts über die Charakterisierungsdaten bezogen auf mehrere verschiedene Arrhythmie-Episoden, und zum Analysieren der Charakterisierungsdaten zum Bereitstellen von Programmierempfehlungen für die Datenerzeugungsvorrichtung, die eine Empfehlung zum Ändern der Speicherparameter für eine Zeitlänge, um die Daten vor der Erfassung eines Tachy-Ereignisses gespeichert werden, enthalten.

2. System nach Anspruch 1, bei der die Episodendaten weiterhin eine/einen ausgewählt aus der Gruppe bestehend aus vorrichtungserzeugten Ereignismarkierern auf dem Elektrointrakardiogramm, Vorrichtungsdiagnosen, während der Arrhythmie-Episode gelieferter Therapie, einem Teil eines zweiten Elektrogramms, Daten von einer elektronischen medizinischen Registrierung oder Daten von einer manuellen Eingabe von Informationen in die Episodendatenbank enthalten.

3. System nach Anspruch 1, bei dem das System eine Entscheidungsdatenbank zum Speichern der von dem Entscheidungsprozessor ausgegebenen Charakterisierungsdaten enthält.

4. System nach Anspruch 1, bei dem die Programmierempfehlung eine aufweist, die ausgewählt ist aus der Gruppe von:
(a) einer Empfehlung zur Verwendung einer Anti-Tachykardie-Schrittgabe (ATP), bei der die Arrhythmie-Klassifizierung MVT ist, wobei MVT vorher mit Schock behandelt wurde, und wobei ATP nicht vorher bei einem Patienten durchgeführt wurde;
(b) einer Empfehlung zum Umschalten zu einem/einer anderen Tachy-Therapiemodus oder -konfiguration, wenn eine ausgegebene Therapie nicht wirksam war; und
(c) einer Empfehlung zur Änderung von Brady-Schrittgabeparametern; und
(d) einer Empfehlung zur Änderung von Erfassungsparametern der Datenerzeugungsvorrichtung, wobei die Programmierempfehlung gewählt ist aus der Gruppe von Ein- oder Ausschalten von morphologiebasierter Unterscheidung und Änderung von Elektrogramm-Erfassungsparametern.

5. System nach Anspruch 1, bei dem der Episodenprozessor konfiguriert ist zum Ausgeben einer Warnung, wenn zumindest eines der Ereignisse in der Gruppe auftritt von:
(a) eine ventrikuläre Antitachykardie-Therapie wurde während einer Arrhythmie-Episode mit einer Arrhythmie-Klassifizierung von supraventrikulärer Tachykardie (SVT) durchgeführt,
(b) eine ventrikuläre Antitachykardie-Therapie wurde während einer Arrhythmie-Episode mit einer Arrhythmie-Klassifizierung von Vorhofflimmern (AF) oder Vorhofflattern (AFL) durchgeführt,
(c) eine ventrikuläre Antitachykardie-Therapie wurde aufgrund von Übererfassung durchgeführt,
(d) ein Schock wurde ohne einen Antitachykardie-Schrittgabeversuch während einer Arrhythmie-Episode mit einer Arrhythmie-Klassifizierung von monomorpher ventrikulärer Tachykardie (MVT) zugeführt, und
(e) ein Schock wurde zu einer hämodynamisch stabilen Arrhythmie-Episode zugeführt.

6. System nach Anspruch 1, bei dem der Episodenprozessor konfiguriert ist zum Ausgeben einer Warnung, die Anweisungen an einen Patienten bereitstellt.

7. System nach Anspruch 1, bei dem der Episodenprozessor konfiguriert ist zum Bereitstellen eines Berichts für einen bestimmten Patienten über die Anzahl von Arrhythmie-Episoden, die als polymorphe ventrikuläre Tachykardie / Kammerflimmern (PVT/VF) klassifiziert sind, und über die Anzahl von Arrhythmie-Episoden, die als MVT klassifiziert sind.

8. System nach Anspruch 1, bei dem der Episodenprozessor konfiguriert ist zum Neuprogrammieren der Datenerzeugungsvorrichtung, um die Programmierempfehlung auszuführen.

9. System nach Anspruch 1, bei dem die Arrhythmie-Klassifizierung ausgewählt ist aus der Gruppe, die weiterhin zumindest aufweist: polymorphe ventrikuläre Tachykardie / Kammerflimmern (PVT/VF), supraventrikuläre Tachykardie (SVT), nodale Wiedereintritts-AV-Tachykardie (AVNRT), Vorhofflimmern (AF), Vorhofflattern (AFL), atriale Tachy, Sinus-Tachy (ST), Doppel-Tachy (DT), schrittgabevermittelte Tachykardie (PMT), eins-zu-eins, Übererfassung, Störungen und Unbestimmtes.

10. System nach Anspruch 1, bei dem die Charakterisierungsdaten eine ausgewählte enthält aus der Gruppe von:
(a) eine Anmerkung, von der ein Teil der Episodendaten die Basis für die Arrhythmie-Episoden-Klassifizierung war;
(b) eine Anmerkung einer Rechtfertigung für die Charakterisierung;
(c) eine Anmerkung eines Moduls eines Entscheidungsprozesses, der zu der Arrhythmie-Episoden-Klassifizierung führt; und
(d) eine Klassifizierung der Arrhythmie-Episode als hämodynamisch stabil oder hämodynamisch instabil.

11. System nach Anspruch 1, weiterhin aufweisend einen Überblicksprozessor, enthaltend eine Benutzerschnittstelle, die konfiguriert ist zum:
Anzeigen zumindest eines bestimmten Teils von Episodendaten für eine der Arrhythmie-Episoden von der Episodendatenbank, und Anzeigen der von dem Entscheidungsalgorithmusprozess ausgegebenen Charakterisierungsdaten; und
Empfangen von Überschreibungscharakterisierungsdaten von dem Benutzer, die die angezeigten Episodendaten charakterisieren.

12. System nach Anspruch 11, bei dem das System konfiguriert ist zum Revidieren der Arrhythmie-Klassifizierung der Arrhythmie-Episode auf der Grundlage der Überschreibungscharakterisierungsdaten.

13. System nach Anspruch 11, bei dem das System konfiguriert ist zum Veranlassen der Revidierung eines Entscheidungsalgorhithmus nach dem Empfangen von Überschreibungscharakterisierungsdaten.

14. Verfahren zum automatischen Entscheiden über Arrhythmie-Episoden-Informationen unter Verwendung eines medizinischen Vorrichtungssystems, welches die Schritte aufweist:
Bereitstellen einer Episodendatenbank mit Episodendaten betreffend mehrere verschiedene Arrhythmie-Episoden, die von zumindest einer Datenerzeugungsvorrichtung des medizinischen Vorrichtungssystem erzeugt wurden, wobei die Datenerzeugungsvorrichtung eine Herzrhythmus-Management-Vorrichtung ist, die in einen Patienten implantierbar ist, wobei die Episodendaten zumindest einen Teil eines Elektrointrakardiogramms und die Ausgabe von einer physiologischen Sensorkonfiguration enthaltend einen oder mehrere Sensoren, die aus der Gruppe bestehend aus einem Drucksensor, einem Beschleunigungsmesser, einem Temperatursensor und einem Impedanzsensor ausgewählt sind, enthalten;
Verarbeiten der Episodendaten für eine der verschiedenen Arrhythmie-Episoden und Ausgeben von Charakterisierungsdaten, die die Episodendaten charakterisieren, wobei die Charakterisierungsdaten eine Arrhythmie-Klassifizierung enthalten, wobei die Arrhythmie-Klassifizierung ausgewählt ist aus einer Gruppe aufweisend zumindest monomorphe ventrikuläre Tachykardie (MVT); und
Verarbeiten der Charakterisierungsdaten, Bereitstellen zumindest eines Berichts über die Charakterisierungsdaten, und Bereitstellen von Programmierempfehlungen für die Datenerzeugungsvorrichtung, enthaltend eine Empfehlung zum Ändern der Speicherparameter für eine Zeitlänge, während der Daten vor der Erfassung eines Tachy-Ereignisses gespeichert werden.

## Revendications

1. Système pour statuer par examen de manière automatique sur une information d'épisode d'arythmie, comprenant :
au moins un dispositif de génération de données, dans lequel le dispositif de génération de données est un dispositif de gestion de rythme cardiaque qui peut être implanté chez un patient ;
une base de données d'épisodes qui comporte des données d'épisode qui concernent une pluralité de différents épisodes d'arythmie et qui sont générées à partir de l'au moins un dispositif de génération de données, dans lequel les données d'épisode incluent au moins une section d'un électrocardiogramme intracardiaque et une sortie en provenance d'une configuration de capteur(s) physiologique(s) qui inclut un ou plusieurs capteur(s) physiologique(s) qui est/sont sélectionné(s) parmi le groupe qui est constitué par un capteur de pression, un accéléromètre, un capteur de température et un capteur d'impédance ;
un processeur pour statuer par examen qui est configuré de manière à ce qu'il reçoive en tant qu'entrée des données d'épisode qui concernent l'un des différents épisodes d'arythmie et de manière à ce qu'il émette en sortie des données de caractérisation qui caractérisent les données d'épisode d'entrée, dans lequel les données de caractérisation incluent une classification d'arythmie, dans lequel la classification d'arythmie est sélectionnée parmi un groupe qui comprend au moins une tachycardie ventriculaire mono-morphique (MVT) ; et
un processeur d'épisode qui est configuré de manière à ce qu'il traite les données de caractérisation et les données d'épisode, de manière à ce qu'il fournisse au moins un rapport portant sur les données de caractérisation qui sont rapportées à une pluralité des différents épisodes d'arythmie et de manière à ce qu'il analyse les données de caractérisation de manière à ce qu'il fournisse des recommandations de programmation pour le dispositif de génération de données, lesquelles recommandations de programmation incluent une recommandation incitant à modifier les paramètres de stockage sur une durée temporelle sur laquelle des données sont stockées avant la détection d'un événement de tachycardie.

2. Système selon la revendication 1, dans lequel les données d'épisode incluent en outre des données qui sont sélectionnées parmi le groupe qui est constitué par des marqueurs d'événement générés par dispositif sur l'électrocardiogramme intracardiaque, des diagnostics de dispositif, une thérapie qui est administrée pendant l'épisode d'arythmie, une section d'un second électrocardiogramme, des données en provenance d'un enregistrement médical électronique et des données en provenance d'une entrée manuelle d'information à l'intérieur de la base de données d'épisodes.

3. Système selon la revendication 1, dans lequel le système inclut une base de données d'appréciations suite à un examen ayant permis de statuer pour stocker les données de caractérisation qui sont émises en sortie par le processeur pour statuer par examen.

4. Système selon la revendication 1, dans lequel la recommandation de programmation comprend une recommandation de programmation qui est sélectionnée parmi le groupe qui est constitué par :
(a) une recommandation qui incite à utiliser une stimulation cardiaque anti-tachycardie (ATP) pour laquelle la classification d'arythmie est MVT, où une MVT a été traitée au préalable à l'aide de l'administration d'un choc et où l'ATP n'a pas été tentée au préalable pour un patient ;
(b) une recommandation qui incite à effectuer une commutation sur un mode ou une configuration de thérapie de tachycardie différent(e) si une thérapie administrée ne s'est pas avérée efficace ;
et
(c) une recommandation qui incite à modifier des paramètres de stimulation cardiaque de bradycardie ; et
(d) une recommandation qui incite à modifier des paramètres de détection du dispositif de génération de données, dans lequel la recommandation de programmation est choisie parmi le groupe qui est constitué par l'activation ou la désactivation de la discrimination basée sur morphologie et par la modification de paramètres de détection d'électrocardiogramme.

5. Système selon la revendication 1, dans lequel le processeur d'épisode est configuré de manière à ce qu'il délivre une alerte si au moins l'un des événements pris parmi le groupe des événements qui suivent est survenu :
(a) une thérapie anti-tachycardie ventriculaire a été administrée pendant un épisode d'arythmie selon une classification d'arythmie constituée par une tachycardie supraventriculaire (SVT) ;
(b) une thérapie anti-tachycardie ventriculaire a été administrée pendant un épisode d'arythmie selon une classification d'arythmie constituée par une fibrillation auriculaire (AF) ou un flutter auriculaire (AFL) ;
(c) une thérapie anti-tachycardie ventriculaire a été administrée du fait d'une sur-détection ;
(d) un choc a été administré sans tentative de stimulation cardiaque anti-tachycardie pendant un épisode d'arythmie selon une classification d'arythmie constituée par une tachycardie ventriculaire mono-morphique (MVT) ; et
(e) un choc a été administré lors d'un épisode d'arythmie hémodynamiquement stable.

6. Système selon la revendication 1, dans lequel le processeur d'épisode est configuré de manière à ce qu'il délivre une alerte qui fournit des instructions à un patient.

7. Système selon la revendication 1, dans lequel le processeur d'épisode est configuré de manière à ce qu'il fournisse un rapport pour un patient particulier, lequel rapport mentionne le nombre d'épisodes d'arythmie qui sont classifiés en tant que tachycardie ventriculaire polymorphique/ fibrillation ventriculaire (PVT/VF) ainsi que le nombre d'épisodes d'arythmie qui sont classifiés en tant que MVT.

8. Système selon la revendication 1, dans lequel le processeur d'épisode est configuré de manière à ce qu'il reprogramme le dispositif de génération de données de manière à ce qu'il exécute la recommandation de programmation.

9. Système selon la revendication 1, dans lequel la classification d'arythmie est sélectionnée parmi le groupe qui comprend en outre au moins une tachycardie ventriculaire polymorphique/fibrillation ventriculaire (PVT/VF), une tachycardie supraventriculaire (SVT), une tachycardie réentrante nodale atrio-ventriculaire - AV - (AVNRT), une fibrillation auriculaire (AF), un flutter auriculaire (AFL), une tachycardie auriculaire, une tachycardie sinusale (ST), une tachycardie double (DT), une tachycardie induite ou entretenue par un stimulateur cardiaque (PMT), un phénomène biunivoque, une sur-détection, un bruit et un état d'indétermination.

10. Système selon la revendication 1, dans lequel les données de caractérisation incluent une information d'annotation ou de classification qui est sélectionnée parmi le groupe qui est constitué par:
(a) une annotation qui est constituée par l'indication de la partie des données d'épisode qui a constitué la base de la classification d'épisode d'arythmie ;
(b) une annotation qui est constituée par une justification pour la caractérisation ;
(c) une annotation qui est constituée par un module d'un processus de décision qui conduit à la classification d'épisode d'arythmie ; et
(d) une classification de l'épisode d'arythmie comme étant hémodynamiquement stable ou hémodynamiquement instable.

11. Système selon la revendication 1, comprenant en outre un processeur de passage en revue, incluant une interface d'utilisateur qui est configurée de manière à ce qu'elle réalise les actions qui suivent :
l'affichage d'au moins une section particulière de données d'épisode pour l'un des épisodes d'arythmie en provenance de la base de données d'épisodes et l'affichage des données de caractérisation qui sont émises en sortie par le processus d'algorithme pour statuer par examen ; et
la réception de données de caractérisation de remplacement par sur-écriture en provenance de l'utilisateur qui caractérisent les données d'épisode affichées.

12. Système selon la revendication 11, dans lequel le système est configuré de manière à ce qu'il réexamine la classification d'arythmie de l'épisode d'arythmie sur la base des données de caractérisation de remplacement par sur-écriture.

13. Système selon la revendication 11, dans lequel le système est configuré de manière à ce qu'il suscite la révision d'un algorithme d'appréciation suite à un examen ayant permis de statuer après la réception de données de caractérisation de remplacement par sur-écriture.

14. Procédé pour statuer par examen de manière automatique sur une information d'épisode d'arythmie en utilisant un système de dispositif médical, comprenant les étapes qui sont constituées par :
la fourniture d'une base de données d'épisodes qui comporte des données d'épisode qui concernent une pluralité de différents épisodes d'arythmie et qui sont générées à partir d'au moins un dispositif de génération de données du système de dispositif médical, dans lequel le dispositif de génération de données est un dispositif de gestion de rythme cardiaque qui peut être implanté sur un patient, dans lequel les données d'épisode incluent au moins une section d'un électrocardiogramme intracardiaque et une sortie en provenance d'une configuration de capteur(s) physiologique(s) qui inclut un ou
plusieurs capteur(s) physiologique(s) qui est/sont sélectionné(s) parmi le groupe qui est constitué par un capteur de pression, un accéléromètre, un capteur de température et un capteur d'impédance; le traitement des données d'épisode qui concernent l'un des différents épisodes d'arythmie et l'émission en sortie de données de caractérisation qui caractérisent les données d'épisode, dans lequel les données de caractérisation incluent une classification d'arythmie, dans lequel la classification d'arythmie est sélectionnée parmi un groupe qui comprend au moins une tachycardie ventriculaire mono-morphique (MVT) ; et
le traitement des données de caractérisation, la fourniture d'au moins un rapport portant sur les données de caractérisation et la fourniture de recommandations de programmation pour le dispositif de génération de données, lesquelles recommandations de programmation incluent une recommandation incitant à modifier les paramètres de stockage sur une durée temporelle sur laquelle des données sont stockées avant la détection d'un événement de tachycardie.
